# EUROPÄISCHE PATENTSCHRIFT

(11) **EP 3 415 527 B1**
(45) Veröffentlichungstag und Bekanntmachung des Hinweises auf die Patenterteilung: **10.12.2025**
(21) Anmeldenummer: 17176043.2
(22) Anmeldetag: 14.06.2017
(51) Int. Cl.: C07K 14/705

(54) **VERWENDUNG DER EXTRAZELLULÄREN DOMÄNE DES TRANSFERRINREZEPTOR 2 ZUR DIAGNOSTIK UND BEHANDLUNG VON PRIMÄR UND SEKUNDÄR SKLEROSIERENDEN ERKRANKUNGEN**
USE OF THE EXTRACELLULAR DOMAIN OF THE TRANSFERRIN RECEPTOR 2 FOR DIAGNOSIS AND TREATMENT OF PRIMARY AND SECONDARY SCLEROSING DISEASES
UTILISATION DU DOMAINE EXTRACELLULAIRE DU RÉCEPTEUR DE TRANSFERT 2 DESTINÉ AU DIAGNOSTIC ET AU TRAITEMENT DES PATHOLOGIES SCLÉROSANTES PRIMAIRES ET SECONDAIRES

(43) Veröffentlichungstag der Anmeldung: 19.12.2018
(73) Patentinhaber: Kymab Ltd., Cambridge CB22 3AT (GB)
(72) Erfinder: Rauner, Martina, 01309 Dresden (DE); Hofbauer, Lorenz C., 01309 Dresden (DE); Platzbecker, Uwe, 01326 Dresden (DE); Baschant, Ulrike, 01277 Dresden (DE)
(74) Vertreter: Zwicker, Jörk

(56) Entgegenhaltungen:
- WO-A1-2015/107075
- WO-A2-00/27874
- WO-A2-2016/039796
- "Abstracts of the ECTS congress 2017 ED - Fielding Roger A; Reid Kieran F", CALCIFIED TISSUE INTERNATIONAL, NEW YORK, NY, US, vol. 100, no. 1, 11 May 2017 (2017-05-11), pages 1 - 174, XP036233897, ISSN: 0171-967X, [retrieved on 20170511], DOI: 10.1007/S00223-017-0267-2

## Beschreibung

Die Erfindung betrifft ein Protein zur Verwendung in der Diagnostik und Behandlung von primär oder sekundär sklerosierenden Erkrankungen, ein Fusionsprotein, eine Nukleotidsequenz und einen Vektor sowie eine pharmazeutische Zusammensetzung zur Verwendung in der Diagnostik und Behandlung von primär oder sekundär sklerosierenden Erkrankungen.

### Stand der Technik

Es gibt zahlreiche sklerosierende Erkrankungen, bei denen es zu einer unkontrollierten Knochenbildung kommt, darunter auch die *Fibrodysplasia ossificans progressive* (FOP). Diese seltene Erkrankung ist durch eine heterotope Ossifikation (HO) gekennzeichnet, welche zur Verknöcherung außerhalb des Skeletts, insbesondere von Muskeln, Sehnen und Weichteilen führt und damit Patienten in ihrer Mobilität stark beeinträchtigt. Menschen mit FOP haben eine mittlere Lebensdauer von 56 Jahren, häufig mit Todesfolge aufgrund der Unfähigkeit des Thorax, eine normale Atmung zu unterstützen. Patienten mit FOP haben eine Mutation im ACVR1-Gen, das für den ACVR1/ALK2-Rezeptor kodiert. Dieser Rezeptor ist Teil des *Bone morphogenetic protein* (BMP)-Signalwegs und ist von entscheidender Bedeutung bei der Regulierung der Knorpel- und Knochenentwicklung. Diese Mutation führt zur erhöhten Aktivität des ACVR1/ALK2-Rezeptors und dadurch zur übermäßigen BMP-Signalgebung, wodurch die gesteigerte und unkontrollierte Knochenbildung resultiert (Shore und Kaplan 2008).

Neben der FOP gibt es andere sklerosierende Erkrankungen, denen unterschiedliche Mechanismen zugrunde liegen. Dazu gehören van Buchem-Syndrom, Sklerosteose, Melorheostose, Pachydermoperiostose, fibröse Dysplasie, Osteochondrodysplasien, Mukopolysaccharidosen, Spondylitis ankylosans, HO nach Traumata, z. B. Gelenkersatzoperationen, Explosionen, Amputationen, Querschnittslähmung, oder nach Kalziphylaxie oder bei malignen Erkrankungen oder Verschleißerkrankungen, z. B. bei Prostatakarzinomen, Nierenzellkarzinomen, tumoröser Kalzinose, Mammakarzinomen, Arthrose oder benignen Läsionen des Knochens. Diese sklerosierenden Erkrankungen sind durch eine unkontrollierte Verknöcherung außerhalb des Skeletts gekennzeichnet.

Herkömmliche Verfahren zur Behandlung sklerosierender Erkrankungen umfassen nichtspezifische Therapien, Steroide, nicht-steroidale Antirheumatika (NSAR), Resektionen oder Bestrahlung (Kölbl *et al.* 2003). Diese Therapien sind jedoch in ihren Anwendungen beschränkt und meist nur symptomlindernd, können allerdings das Fortschreiten der Erkrankung nicht aufhalten. Nach einer Resektion beträgt beispielsweise die Wahrscheinlichkeit einer wiederkehrenden HO bis zu 80%. Insbesondere Steroide bewirken eine Hemmung der Knochenbildung, weisen jedoch einer Reihe von Nebenwirkungen auf, wie Adipositas, Diabetes, spröde Haut oder Muskelschwund.

### Aufgabe der Erfindung

Daher besteht die Aufgabe der vorliegenden Erfindung darin, ein Medikament zur Behandlung von sklerosierenden Erkrankungen bereitzustellen.

Ferner ist es Aufgabe der Erfindung, ein Medikament bereitzustellen, welches weniger Nebenwirkungen als bekannte Behandlungsmethoden hat.

### Wesen der Erfindung

Die vorliegende Erfindung betrifft den Gegenstand der Ansprüche 1 bis 16.

Erfindungsgemäß wird die Aufgabe gelöst durch das Protein mit einer Aminosäuresequenz mit mindestens 70% Identität zu der Sequenz SEQ ID NO. 1 oder deren Fragmente zur Verwendung in der Diagnostik und Behandlung von primär oder sekundär sklerosierenden Erkrankungen.

Unter Identität wird die Anzahl an übereinstimmenden Aminosäuren bezogen auf die Gesamtanzahl an Aminosäuren verstanden.

Unter einem Fragment wird ein Teil der Aminosäuresequenz des erfindungsgemäßen Proteins verstanden, bevorzugt ein Fragment bestehend aus der PA-Domäne (SEQ ID NO. 5); ein Fragment bestehend aus der Peptidase-M28-Domäne (SEQ ID NO. 6) oder ein Fragment bestehend aus der Tfr-ähnlichen Dimerisierungsdomäne (SEQ ID NO. 7).

Unter primär oder sekundär sklerosierenden Erkrankungen werden Erkrankungen verstanden, bei denen es zur Ossifikation von Weichteilgewebe kommt, wobei die Sklerose als primäre oder sekundäre Folge der Erkrankung auftritt.

Primär oder sekundär sklerosierenden Erkrankungen umfassen *Fibrodysplasia ossificans progressiva* (FOP), van Buchem-Syndrom, Sklerosteose, Melorheostose, Pachydermoperiostose, fibröse Dysplasie, Osteochondrodysplasien, Mukopolysaccharidose, Spondylitis ankylosans, heterotope Ossifikation (HO) nach Traumata, bevorzugt bei Sklerosierungen nach Gelenkersatzoperationen, Explosionen, Amputationen, Querschnittslähmung, Kalziphylaxie oder bei malignen Erkrankungen oder Verschleißerkrankungen, besonders bevorzugt bei Prostatakarzinomen, Nierenzellkarzinomen, tumoröser Kalzinose, Mammakarzinomen, Arthrose und benignen Läsionen des Knochens.

In einer bevorzugten Ausführungsform erfolgt die Verwendung in der Diagnostik und Behandlung von heterotoper Ossifikation (HO) oder *Fibrodysplasia ossificans progressiva* (FOP).

Unter HO, auch *Myositis ossificans,* wird eine Erkrankung verstanden, bei der eine Verknöcherung des Weichteilgewebes außerhalb des Skelettsystems als Folge einer Gewebsverletzung erfolgt.

Unter *Fibrodysplasia ossificans progressiva* (FOP), auch *Fibrodysplasia ossificans multiplex progressiva, Myositis ossificans progressiva* oder Münchmeyer-Syndrom, wird eine genetische Erkrankung verstanden, bei der eine fortschreitende Verknöcherung des Binde- und Stützgewebes des menschlichen Körpers erfolgt.

Das Protein mit einer Aminosäuresequenz SEQ ID NO. 1 kann aus dem humanen Transferrinrezeptor (Tfr) 2α, dem humanen Transferrinrezeptor (Tfr) 2β, bevorzugt der extrazellulären Domäne von humanen Tfr2α, isoliert werden.

Das erfindungsgemäße Protein bindet Mitglieder der *transforming growth factor*-β (TGF-β)/knochenmorphogenetische Proteine (*bone morphogenetic proteins,* BMP)-Familie, bevorzugt BMPs, besonders bevorzugt BMP-2, BMP-4, BMP-6 und BMP-7.

Unter *transforming growth factor*-β (TGF-β)/knochenmorphogenetische Proteine (*bone morphogenetic proteins,* BMP)-Familie wird eine Gruppe ähnlicher Signalproteine verstanden, welche Mitglieder der TGF-β-Rezeptorfamilie binden. Die TGF-β/BMP-Familie umfasst TGFβ1, TGFβ2, TGFß3, BMPs, *growth differentiation factors* (GDFs), Activin und Inhibin, Myostatin, Anti-Müller-Hormon (AMH), und Nodal.

Unter *bone morphogenetic proteins* (BMPs) wird eine Gruppe parakriner Signalproteine verstanden, welche BMP-Rezeptoren binden. In einer Ausführungsform, sind BMPs ausgewählt aus BMP1, BMP2, BMP3, BMP4, BMP5, BMP6, BMP7, BMP8a, BMP8b, BMP-9, BMP10 oder BMP15, bevorzugt BMP-2, BMP-4, BMP-6 oder BMP-7.

Vorteilhaft wird bei der Behandlung von primär oder sekundär sklerosierenden Erkrankungen durch das erfindungsgemäße Protein der BMP-Signalweg und damit die Knochenbildung spezifisch gehemmt.

In einer Ausführungsform erfolgt die Diagnostik von primär oder sekundär sklerosierenden Erkrankungen mittels des erfindungsgemäßen Proteins durch Detektion von Mitgliedern der TGF-β/BMP-Familie, bevorzugt BMPs, besonders bevorzugt BMP-2, BMP-4, BMP-6 und BMP-7.

In einer Ausführungsform erfolgt die Diagnostik von primär oder sekundär sklerosierenden Erkrankungen mittels des erfindungsgemäßen Proteins im Blut, im Blutplasma, im Blutserum oder in Gewebe. In einer bevorzugten Ausführungsform ist das Gewebe Knochen oder Knorpel. Unter Blutplasma wird der flüssige Bestandteil des Bluts verstanden. Unter Blutserum wird das Blutplasma ohne Gerinnungsfaktoren verstanden.

In einer Ausführungsform erfolgt die Diagnostik von primär oder sekundär sklerosierenden Erkrankungen mittels des erfindungsgemäßen Proteins durch einen Immunassay. Unter einem Immunassay werden Nachweisverfahren verstanden, bei denen ein Analyt in einer flüssigen Phase durch eine Antigen-Antikörper-Bindung nachgewiesen wird.

In einer Ausführungsform ist der Immunassay ausgewählt aus einem *Enzyme-linked Immunosorbent Assay* (ELISA) oder *Enzyme-linked Immuno Spot Assay* (ELIspot-Assay). Unter einem ELISA wird ein Antikörper-basiertes Nachweisverfahren verstanden, welches auf einer enzymatischen Farbreaktion basiert. Unter einem ELIspot-Assay wird ein Nachweisverfahren zur Detektion von Antikörpern verstanden, welche von Immunzellen nach Stimulation mit Antigenen sezerniert und an einer Membran immobilisiert werden.

In einer Ausführungsform erfolgt die Diagnostik von primär oder sekundär sklerosierenden Erkrankungen zur Abschätzung der Prognose der Erkrankung, zur Abschätzung des Ansprechens auf eine Behandlung und/oder zur Risikostratifizierung. Unter einer Risikostratifizierung wird das Abschätzen des Risikos, mit dem eine Erkrankung fortschreitet, zu Komplikationen oder zum Tod führt, verstanden.

In einer Ausführungsform umfasst das erfindungsgemäße Protein zur Verwendung in der Diagnostik und Behandlung von primär oder sekundär sklerosierenden Erkrankungen Sequenz SEQ ID NO. 1 oder SEQ ID NO. 2.

Das Protein mit einer Aminosäuresequenz SEQ ID NO. 2 kann aus dem murinen Transferrinrezeptor (Tfr) 2α, dem murinen Transferrinrezeptor (Tfr) 2β, bevorzugt der extrazellulären Domäne von murinen Tfr2α, isoliert werden.

In einer Ausführungsform weist das erfindungsgemäße Protein 232 Aminosäuren bis 801 Aminosäuren, bevorzugt 487 Aminosäuren bis 801 Aminosäuren auf, besonders bevorzugt 600 Aminosäuren bis 750 Aminosäuren.

In einer Ausführungsform ist das erfindungsgemäße Protein zur Verwendung in der Diagnostik und Behandlung von primär oder sekundär sklerosierenden Erkrankungen der humane Transferrinrezeptor (Tfr) 2α (SEQ ID NO. 3), der murine Transferrinrezeptor (Tfr) 2α (SEQ ID NO. 4), der humane Transferrinrezeptor (Tfr) 2β (SEQ ID NO. 1) oder die extrazelluläre Domäne von humanen Tfr2α (SEQ ID NO. 1), der murine Transferrinrezeptor (Tfr) 2β (SEQ ID NO. 2) oder die extrazelluläre Domäne von murinen Tfr2α (SEQ ID NO. 2).

Gegenstand der Erfindung ist auch ein Fusionsprotein umfassend mindestens ein erfindungsgemäßes Protein zur Verwendung in der Diagnostik und Behandlung von primär oder sekundär sklerosierenden Erkrankungen.

In einer Ausführungsform umfasst das Fusionsprotein mindestens einen Protein-Tag. In einer Ausführungsform ist der mindestens eine Protein-Tag ausgewählt aus einem poly-Histidin (His)-Tag, Glutathion-S-Transferase (GST)-Tag, *maltose binding protein* (MBP)-Tag, Myc-Tag, Streptavidin (Strep)-Tag oder einem Farbstoff, bevorzugt einem Fluoreszenzfarbstoff, besonders bevorzugt ein *green fluorescent protein* (GFP) oder ein *yellow fluorescent protein* (YFP).

In einer Ausführungsform weist das erfindungsgemäße Protein oder das Fusionsprotein umfassend mindestens ein erfindungsgemäßes Protein mindestens eine Modifizierung auf.

In einer Ausführungsform, ist die mindestens eine Modifizierung ausgewählt aus Proteinen enthaltend D-Aminosäuren, Pseudopeptidbindungen, Aminoalkoholen, nicht-proteinogenen Aminosäuren, Aminosäuren mit modifizierten Seitengruppen und/oder zirkularisierten Proteinen. Vorteilhaft weisen Proteine mit Modifizierungen eine erhöhte Stabilität auf.

In einer Ausführungsform wird das erfindungsgemäße Protein oder das Fusionsprotein umfassend mindestens ein erfindungsgemäßes Protein in der Diagnostik von Mitgliedern der TGF-β/BMP-Familie oder in der Diagnostik von Erkrankungen mit erhöhter BMP-Rezeptoraktivierung verwendet.

Unter einer erhöhten BMP-Rezeptoraktivierung wird eine Aktivierung mindestens eines BMP-Rezeptors verstanden, welche durch eine Mutation eines BMP-Rezeptors verursacht wird (Shore und Kaplan 2008), bevorzugt konstitutiv aktivierende Mutationen. Unter konstitutiv aktivierenden Mutationen werden Mutationen verstanden, bei denen mindestens ein BMP-Rezeptor in Abwesenheit von BMPs aktiviert ist.

In einer Ausführungsform wird das erfindungsgemäße Protein oder das Fusionsprotein umfassend mindestens ein erfindungsgemäßes Protein in der Behandlung von Erkrankungen mit erhöhter BMP-Rezeptoraktivierung verwendet.

Gegenstand der Erfindung ist auch eine Nukleotidsequenz umfassend eine Sequenz kodierend für ein erfindungsgemäßes Protein oder ein Fusionsprotein umfassend mindestens ein erfindungsgemäßes Protein.

In einer Ausführungsform umfasst die Nukleotidsequenz SEQ ID NO.8 oder SEQ ID NO. 9.

Ein weiterer Aspekt der Erfindung betrifft einen Vektor umfassend eine Nukleotidsequenz umfassend eine Sequenz kodierend für ein erfindungsgemäßes Protein oder ein Fusionsprotein umfassend mindestens ein erfindungsgemäßes Protein.

Unter einem Vektor wird Nukleinsäureträger zur Übertragung einer Nukleinsäure in eine Zelle durch Transfektion oder Transduktion verstanden. In einer Ausführungsform sind Vektoren ausgewählt aus Plasmiden, viralen Vektoren oder anderen Nukleinsäureträgern, welche eine Nukleotidsequenz umfassend eine Sequenz kodierend für ein erfindungsgemäßes Protein oder ein Fusionsprotein umfassend mindestens ein erfindungsgemäßes Protein durch genetische Rekombination (rekombinant) enthalten.

Gegenstand der Erfindung ist auch eine pharmazeutische Zusammensetzung umfassend mindestens ein erfindungsgemäßes Protein oder ein Fusionsprotein umfassend mindestens ein erfindungsgemäßes Protein.

In einer Ausführungsform ist die pharmazeutische Zusammensetzung eine Lösung, Tablette oder Kapsel. In einer Ausführungsform wird das erfindungsgemäße Protein als Beschichtung für Implantatmaterialien, bevorzugt Metalle oder Kunststoffe; und/oder Implantate, bevorzugt Prothesen, Schrauben oder Nägel; verwendet.

In einer Ausführungsform wird die pharmazeutische Zusammensetzung lokal intraartikulär, intramuskulär oder systemisch subkutan, intravenös oder über eine orale Gabe verabreicht. In einer Ausführungsform ist die pharmazeutische Zusammensetzung in einer passenden Form für die intraartikuläre, intramuskuläre, subkutane, intravenöse oder orale Verabreichung.

In einer Ausführungsform, enthält die pharmazeutische Zusammensetzung das erfindungsgemäße Protein oder das Fusionsprotein umfassend mindestens ein erfindungsgemäßes Protein in einer Dosis von 10 µg/kg bis 100 mg/kg Körpergewicht pro Verabreichung.

In einer weiteren Ausführungsform enthält die pharmazeutische Zusammensetzung weiterhin ein pharmazeutisch akzeptables Verdünnungsmittel oder Trägermaterial. In einer Ausführungsform ist das pharmazeutisch akzeptable Verdünnungsmittel oder Trägermaterial eine wässrige Lösung, bevorzugt eine gepufferte wässrige Lösung, eine wässrige Salzlösung oder wässrige Glycinlösung. In einer Ausführungsform ist die gepufferte wässrige Lösung ausgewählt aus einer Histidin-gepufferten wässrigen Lösung mit einem pH-Wert von pH 5,0 bis pH 7,0, oder einer Natriumsuccinat-, Natriumcitrat-, Natriumphosphat-, oder Kaliumphosphat-gepufferten wässrigen Lösung. In einer Ausführungsform hat die gepufferte wässrige Lösung eine Konzentration von 1 mmol/l (mM) bis 500 mM, bevorzugt 1 mM bis 50 mM. In einer weiteren Ausführungsform, umfasst das pharmazeutisch akzeptable Verdünnungsmittel oder Trägermaterial Natriumchlorid, bevorzugt in einer Konzentration zwischen 0 mM und 300 mM, besonders bevorzugt in einer Konzentration von 150 mM.

In einer Ausführungsform, umfasst die pharmazeutische Zusammensetzung weiterhin mindestens einen pharmazeutisch akzeptablen Hilfsstoff. Unter einem "Hilfsstoff" wird eine Verbindung verstanden, welche physiologische Bedingungen, hinsichtlich des pH-Werts und/oder der Ionenstärke, einstellt und/oder die Stabilität der pharmazeutischen Zusammensetzung erhöht. In einer Ausführungsform ist der mindestens eine pharmazeutisch akzeptable Hilfsstoff ausgewählt aus Natriumacetat, Natriumchlorid, Kaliumchlorid, Calciumchlorid oder Natriumlactat.

In einer Ausführungsform ist die pharmazeutische Zusammensetzung steril. Die pharmazeutische Zusammensetzung wird durch bekannte Methoden sterilisiert.

Ein weiterer Aspekt der Erfindung betrifft die Verwendung der pharmazeutischen Zusammensetzung in der Diagnostik und Behandlung von primäroder sekundär sklerosierenden Erkrankungen.

In einer Ausführungsform erfolgt die Verwendung der pharmazeutischen Zusammensetzung zur Verabreichung an einen Probanden. Unter einem Probanden wird ein Individuum oder ein Patient verstanden. In einer Ausführungsform ist der Proband ausgewählt aus Menschen oder Tieren. In einer Ausführungsform sind ausgewählt aus Nagetieren, bevorzugt Mäuse, Ratten, Hamster oder Meerschweinchen; Hunden, Kaninchen, Farmtiere, bevorzugt Ziegen, Schafe, Schweine; und nicht-humane Primaten, bevorzugt Schimpansen, Orang-Utans oder Gorillas.

In einer Ausführungsform erfolgt die Verwendung der pharmazeutischen Zusammensetzung in der Diagnostik von Mitgliedern der TGF-β/BMP-Familie und Behandlung von Erkrankungen mit erhöhter BMP-Rezeptoraktivierung.

Gegenstand der Erfindung ist auch ein Verfahren zur Diagnostik und/oder Behandlung von primär oder sekundär sklerosierenden Erkrankungen umfassend die Verabreichung des erfindungsgemäßen Proteins und/oder der pharmazeutischen Zusammensetzung.

In einer Ausführungsform erfolgt die Diagnostik und/oder Behandlung von primär oder sekundär sklerosierenden Erkrankungen an Menschen.

Für die Diagnostik und/oder Behandlung wird eine sterile pharmazeutische Zusammensetzung, enthaltend eine pharmakologisch wirksame Dosismenge eines oder mehrerer erfindungsgemäßer Proteine einem Patienten verabreicht, um primär oder sekundär sklerosierenden Erkrankungen zu diagnostizieren und/oder behandeln.

In einer Ausführungsform erfolgt die Verabreichung lokal, bevorzugt als intraartikuläre oder intramuskuläre Injektion; oder systemisch, bevorzugt als subkutane, intramuskuläre oder intravenöse Injektion oder Infusion, oder über eine orale oder transdermale Gabe.

Neben dem Einsatz in der Diagnostik und/oder Behandlung von primär oder sekundär sklerosierenden Erkrankungen eignen sich die erfindungsgemäßen Proteine für die biologische Forschung und andere Anwendungen, in denen der Nachweis eines Mitgliedes der TGF-β/BMP-Familie interessant ist. Derartige Anwendungen sind insbesondere *Western Blot,* Immunfärbungen von Zellen (z. B. für die Durchflusszytometrie und Mikroskopie) und ELISA, sowie der Einsatz als Tracer in bildgebenden Techniken wie z. B. der CT (Computer-Tomographie), PET/CT (Positronen-Emission-Tomographie).

Für die Realisierung der Erfindung ist es auch zweckmäßig, die vorbeschriebenen Ausführungsformen und Merkmale der Ansprüche zu kombinieren.

### Ausführungsbeispiele

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

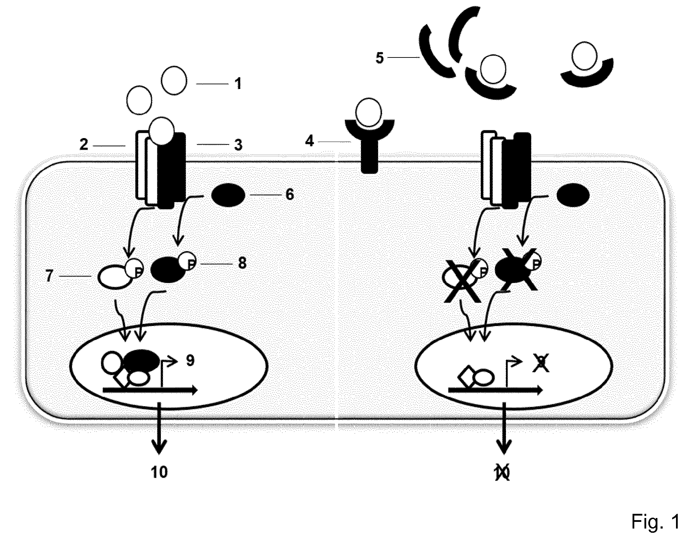

Es zeigen die
**Fig. 1** Schema des Einflusses von BMPs (1) auf die Knochenbildung (links). BMPs binden BMP-Rezeptoren (BMPR-I (2) oder BMPR-II (3)), wodurch eine Signalkaskade (Phosphorylierung (8) von Smad-Protein (6) und MAP-Kinase (7)) ausgelöst wird, welche die Knochenbildung (10) durch die Expression von Osteoblastengenen (9) aktiviert. Schema der Bindung von BMPs (1) an Tfr2α (4) (mittig). Schema des Einflusses des erfindungsgemäßen Proteins (5) auf die Knochenbildung (10) (rechts). Die erfindungsgemäßen Proteine (5) binden BMPs (1), wodurch keine Bindung an BMP-Rezeptoren (BMPR-I (2) oder BMPR-II (3)) erfolgt und die Knochenbildung nicht aktiviert wird.

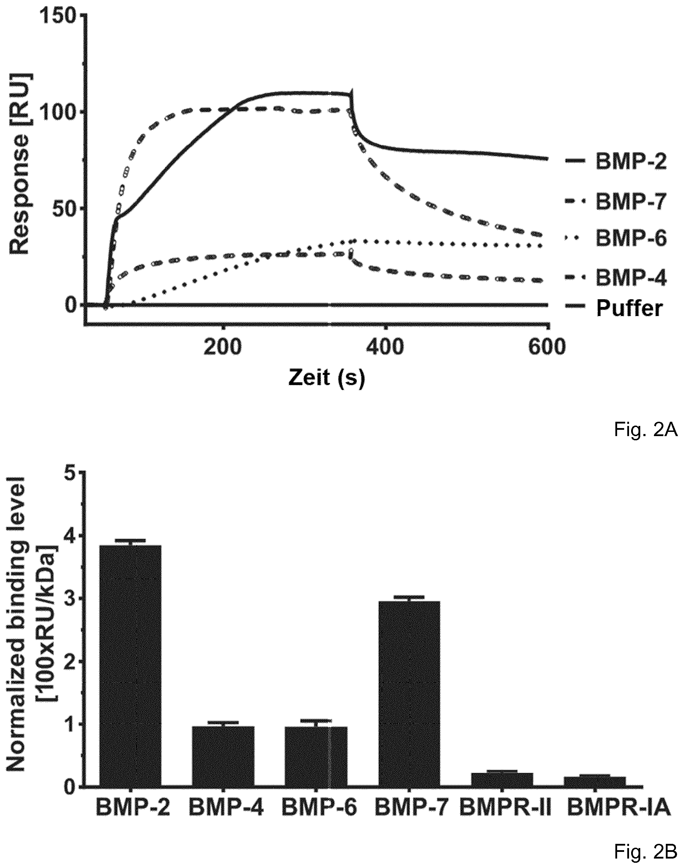

**Fig. 2** SPR-Messungen der Bindung von BMPs und den erfindungsgemäßen Proteinen. **A** Bindung von BMP-2, BMP-4, BMP-6 und BMP-7 an Tfr2-ECD. **B** Quantifizierung der Bindungslevel bezogen auf die Molmasse von BMP-2, BMP-4, BMP-6 und BMP-7 an Tfr2-ECD im Vergleich zu dem Bindungslevel von BMPR-II und BMPR-IA.

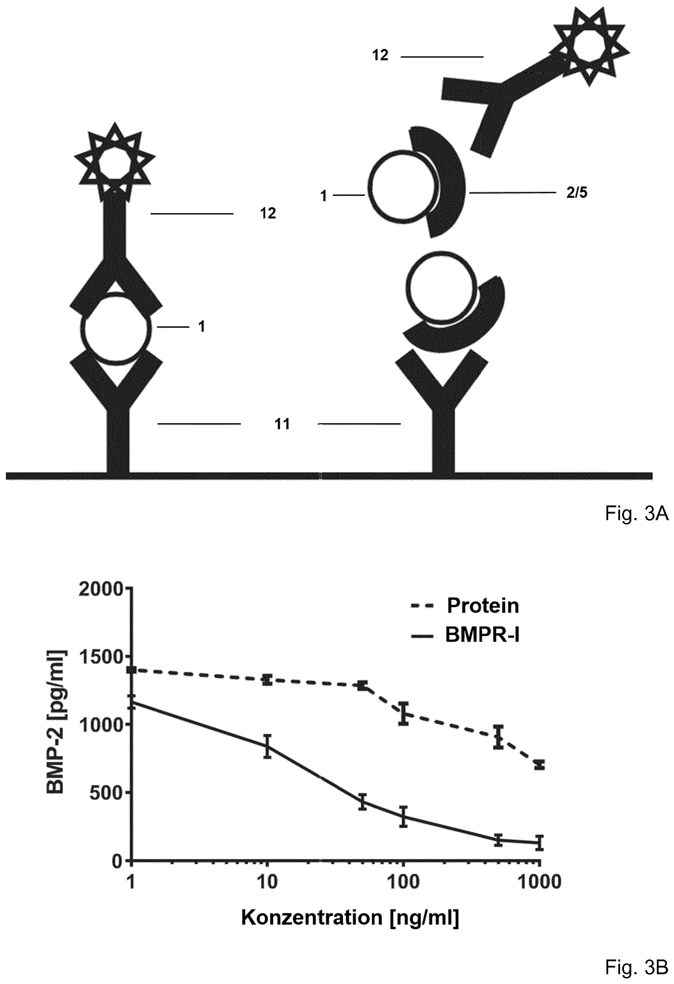

**Fig. 3** **A** Schema des BMP-2-kompetitiven ELISA (*enzyme linked immunosorbent assay*): Signal durch die Bindung von BMPs (1), insbesondere BMP-2, an den Fängerantikörper (11) und Bindung des Detektionsantikörpers (12) an BMP-2 (links). Verringertes Signal aufgrund der Bindung des erfindungsgemäßen Proteins (5) bzw. BMPR-I (2) an BMP-2, wodurch keine Bindung des Fängerantikörpers (11) und Detektionsantikörpers (12) erfolgt (rechts). **B** BMP-2-kompetitiver ELISA: Einfluss von der Konzentration des erfindungsgemäßen Proteins bzw. BMPR-I auf das Signal des BMP-2-Detektionsantikörpers bei gleichbleibender BMP-2-Konzentration.

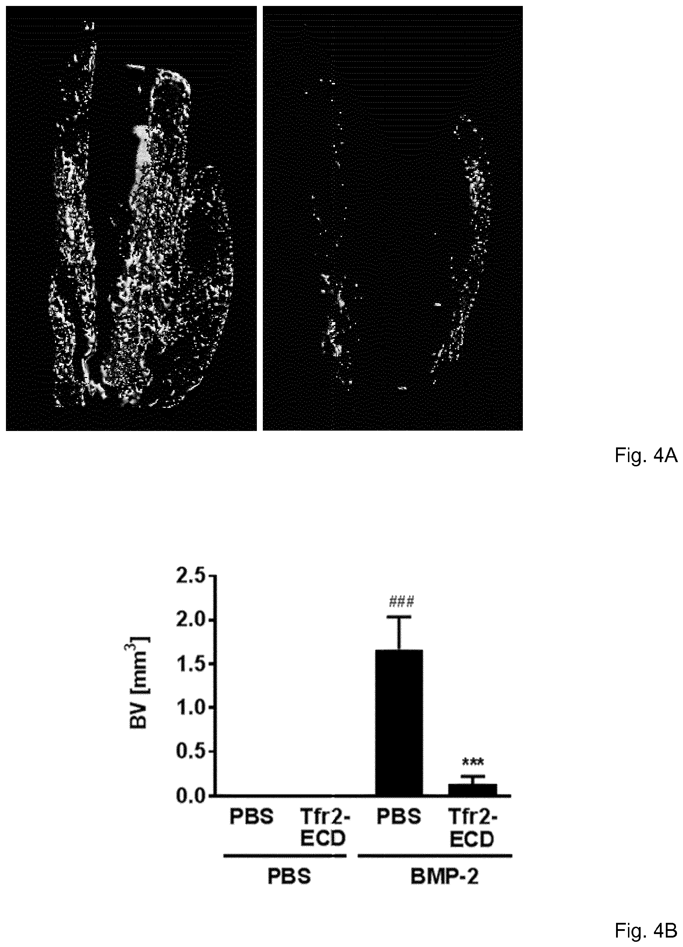

**Fig. 4** die Hemmung der HO in Mäusen (C57BL/6-Mäusen) durch das erfindungsgemäße Protein, insbesondere Tfr2-ECD, über die Bindung von BMP-2. **A** und **B** zeigen die Mineralisierung durch Bestimmung des Knochenvolumens mittels µCT (Mikrotomographie). **A** zeigt die CT-Aufnahmen der Knochenbildung bei der Applikation von BMP-2 bzw. bei der Applikation von BMP-2 zusammen mit Tfr2-ECD. **B** zeigt die Quantifizierung des Knochenvolumens. PBS dient als Negativkontrolle. Bei der Applikation von BMP-2 zeigt sich eine Erhöhung des Knochenvolumens nach zwei Wochen. Die Applikation von BMP-2 zusammen mit Tfr2-ECD zeigt eine signifikante Reduktion der Knochenbildung im Vergleich zu BMP-2 als Referenz (Mittelwert ± Standardabweichung; n = 3 - 6 pro Gruppe; ***p < 0,001 in Bezug zu PBS (Kontrolle).

### Herstellung der Tfr2 extrazellulären Domäne (Tfr2-ECD)

Die Nukleinsäuresequenz der gesamten murinen extrazellulären Domäne (ECD, aa 103-798) von Tfr2 inklusive eines 6x-His-Tags erfolgt durch Genscript (Germany). Die rekombinante His-Tfr2-ECD wird in Sf9-Insektenzellen exprimiert unter Verwendung des Baculovirusexpressionsystems (pOCC211-Tfr2-ECD). Zellkulturüberstände werden gesammelt und mittels einer HisTrap-Säule gereinigt. Nach Waschschritten mit Phosphat-gepufferter Salzlösung *(phosphate buffered saline,* PBS) wird das His-Tfr2-ECD-Protein mittels PBS mit Imidazol eluiert.

### Oberflächenplasmonresonanzmessung (Surface plasmon resonance, SPR)

Die Wechselwirkungen zwischen Tfr2-ECD mit BMPs (BMP-2, -4, -6, -7 von R&D Systems) und BMP-Rezeptoren (BMPR-IA, BMPR-II von R&D Systems) werden mittels Biacore T100 (GE Healthcare) analysiert.

Dazu wird Tfr2-ECD auf einen Series S Sensor Chip C1 (GE Healthcare) durch Kopplung der Aminogruppen bei 25 °C immobilisiert. Die Carboxylgruppen auf der Chipoberfläche werden für für 7 min mit einer Mischung aus 196 mM 1-Ethyl-3-(3-dimethylaminopropyl)-carbodiimid-hydrochlorid und 50 mM N-Hydroxysuccinimid bei einer Flussrate von 10 µl/min aktiviert. Danach erfolgt die Injektion von 5 µg/ml Tfr2-ECD, verdünnt mit Natriumacetatpuffer (pH 4,5), mit einer Flussrate von 5 µl/min bis zu einer relativen Belegung von 200 RU. Nicht reagierte Gruppen werden durch die Injektion von 1 M Ethanolamin-HCl (pH 8,5) für 7 min mit einer Flussrate von 10 µl/min inaktiviert. Zur Herstellung einer Referenzoberfläche erfolgt das gleiche Vorgehen ohne die Injektion von Tfr2-ECD.

Die Bindungsanalysen werden bei 37 °C mit einer Flussrate 30 µl/min durchgeführt. Jeder Analyt wird mit Laufpuffer (HBS-P, pH 7,4 mit 50 nM FeCl₃) verdünnt. BMPs werden mit einer Konzentration von 50 nM und BMP-Rezeptoren mit einer Konzentration von 200 nM verwendet. Die Bindungsanalysen werden mit einer Analytinjektion für 300 s über die Tfr2-ECD-Oberfläche, gefolgt von einer Dissoziation für 1000 s durchgeführt. Die Werte der Bindungslevel werden 10 s vor dem Ende der Injektion in Bezug zur Basislinie abgelesen und bezüglich der Molmasse korrigiert. Nach der Dissoziation für 1000 s wird die Chipoberfläche mit HBS-P mit 5 M NaCl und 50 mM NaOH für 60 s regeneriert und für 1000 s stabilisiert. Die Bindungsparameter werden mittels der Biacore^{™} T100-Auswertungssoftware 2.03 ermittelt.

**Fig. 2** zeigt die Bindung des erfindungsgemäßen Proteins an verschiedene BMP-Liganden (BMP-2, BMP-4, BMP-6, BMP-7).

### BMP-2-kompetitiver ELISA (enzyme linked immunosorbent assay)

Zur Durchführung des BMP-2-kompetitiven ELISA wird das Duo Set BMP-2 ELISA-Kit von R&D Systems verwendet. Nach der Beschichtung der Platte mit BMP-2-Fängerantikörper über Nacht wird 1,5 ng/ml BMP-2 zusammen mit ansteigenden Konzentrationen von Tfr2-ECD oder BMPR-IA (Positivkontrolle, R&D Systems) zum Assay hinzugefügt. Nach einer Inkubation für 1 h bei Raumtemperatur und intensivem Waschen wird der Detektionsantikörper entsprechend der Herstellerangaben zugegeben und die Menge an nicht an Tfr2-ECD oder BMPR-IA gebundenem, am Fänger- und Detektionsantikörper-gebundenen BMP-2 quantifiziert.

**Fig. 3** zeigt die Bindung des erfindungsgemäßen Proteins an BMPs, insbesondere BMP-2. Mit zunehmender Konzentration an erfindungsgemäßen Protein nimmt das Signal des BMP-2-Detektionsantikörpers trotz gleicher BMP-2-Konzentration ab. Der Verlauf ist vergleichbar mit der Bindung von BMP-2 mit dem BMP-Rezeptor I (BMPR-I).

### Mausmodell der heterotopen Ossifikation (HO)

Männliche und weibliche C57BL/6-Mäuse werden für das Modell der HO verwendet. Die HO wird durch die Injektion von BMP-2 in den Muskel ausgelöst (Wosczyna *et al.* 2012).

Sämtliche Mäuse werden mit einer Standardernährung mit Wasser *ad libitum* gefüttert und in Gruppen von fünf Mäusen pro Käfig gehalten. Die Mäuse werden einem 12 h hell/dunkel-Zyklus und einer Luftkühlung auf 23 °C ausgesetzt (kein spezieller Pathogen-freier Raum). Bereicherung erfolgt in Form von Pappkartonhäusern und Einstreumaterial. Die Mäuse werden zufällig in die verschiedenen Behandlungsgruppen aufgeteilt und anschließend die Analysen als Blindstudie durchgeführt.

Die Untersuchung der HO erfolgt durch Behandlung mit 2,5 µl einer 1 mg/ml rekombinantem BMP-2-Lösung (Thermo Fisher Scientific) oder 2,5 µl einer 1 mg/ml Tfr2-ECD gemischt mit 47,5 µl Matrigel (BD Bioscience) bei 0 °C. Für die Kombinationsbehandlung wird 2,5 µl einer 1 mg/ml rekombinantem BMP-2-Lösung mit 2,5 µl einer 1 mg/ml Tfr2-ECD und 45 µl Matrigel gemischt.

Die Matrigelmischungen werden in den *musculus tibialis anterior* von 10 Wochen alten, weiblichen Wildtyp-Mäusen injiziert. Nach zwei Wochen werden die Beine untersucht.

### µCT (Mikrotomographie) und Knochenmikromineralisierungsdichte

Die Knochenmikroarchitektur wird mittels vivaCT40 (Scanco Medical, Schweiz) analysiert. Der gesamte Unterschenkelknochen wird mit einer Auflösung von 10,5 µm mit einer Röntgenstrahlung von 70 kVp, 114 mA und einer Integrationszeit von 200 ms gemessen. Für die Auswertung des Knochens werden vordefinierte Skripte von Scanco verwendet (#1).

Das Mausmodell der HO entwickelt eine BMP-2 induzierte Verknöcherung des Muskelgewebes. **Fig. 4** zeigt die Hemmung der Verknöcherung bzw. der HO in Mäusen (C57BL/6-Mäusen) durch Tfr2-ECD über die Bindung von BMP-2.

### Statistische Analyse

Die Daten werden als Mittelwert ± Standardabweichung (*standard deviation*, SD) angegeben. Graphen und Statistiken werden mittels Graphpad Prism 6.0-Software erstellt. Die Normalität der Daten wird mittels Kolmogorow-Smirnov-Test bestimmt. Im Falle einer Normalverteilung werden statistische Auswertungen mit Zweistichprobenvergleich unter Verwendung des Zweistichproben-Student's T-Test durchgeführt. Eine einseitige Varianzanalyse (ANOVA) wird für Experimente mit mehr als zwei Gruppen verwendet. Eine zweiseitige ANOVA mit Bonferroni Posthoc-Test wird zur Analyse der Behandlungseffekte verwendet. Falls Daten nicht der Normalverteilung entsprechen, werden der Mann-Whitney-Test und der Wilcoxon-Vorzeichen-Rang-Test zur Datenanalyse verwendet.

### Zitierte Nichtpatentliteratur

Shore EM, Kaplan FS (2008) Insights from a rare genetic disorder of extra-skeletal bone formation, fibrodysplasia ossificans progressiva (FOP). Bone 43: 427-433.
Kölbl O, Barthel T, Krödel A, Seegenschmiedt MH (2003) Prävention von heterotopen Ossifikationen nach Totalendoprothese des Hüftgelenks. Deutsches Ärzteblatt 45: 2944-2954.
Roetto, A. et al. Comparison of 3 Tfr2-deficient murine models suggests distinct functions for Tfr2-alpha and Tfr2-beta isoforms in different tissues. Blood 115, 3382-3389, doi:10.1182/blood-2009-09-240960 (2010).
Wosczyna MW, Biswas AA, Cogswell CA, and Goldhamer DJ (2012) Multipotent Progenitors Resident in the Skeletal Muscle Interstitium Exhibit Robust BMP-Dependent Osteogenic Activity and Mediate Heterotopic Ossification. J Bone Miner Res 27: 1004-1017.

### Bezugszeichen

- 1: BMP
- 2: BMPR-I
- 3: BMPR-II
- 4: Tfr2α
- 5: Protein
- 6: Smad-Protein
- 7: MAP-Kinase
- 8: Phosphorylierung
- 9: Osteoblastengene
- 10: Knochenbildung
- 11: Fängerantikörper
- 12: Detektionsantikörper

## Patentansprüche

1. Protein oder ein Fragment davon mit einer Aminosäuresequenz mit mindestens 70% Identität zu der Sequenz SEQ ID NO. 1 zur Verwendung in der Behandlung von primär oder sekundär sklerosierenden Erkrankungen, wobei das Protein oder das Fragment davon Mitglieder der transforming growth factor-β (TGF-β)/knochenmorphogenetische Protein-Familie bindet.

2. Protein umfassend Sequenz SEQ ID NO. 1 oder SEQ ID NO. 2 zur Verwendung in der Behandlung von primär oder sekundär sklerosierenden Erkrankungen.

3. Protein zur Verwendung nach Anspruch 1 oder 2 mit einer Länge von 232 Aminosäuren bis 801 Aminosäuren.

4. Protein zur Verwendung nach einem der Ansprüche 1 bis 3, **dadurch gekennzeichnet, dass** das Protein ein Transferrinrezeptor (Tfr) 2α, ein Transferrinrezeptor (Tfr) 2β oder eine extrazelluläre Domäne von Tfr2α ist.

5. Protein zur Verwendung nach einem der Ansprüche 1 bis 4, **dadurch gekennzeichnet, dass** das Protein der humane Transferrinrezeptor (Tfr) 2α (SEQ ID NO. 3), der murine Transferrinrezeptor (Tfr) 2α (SEQ ID NO. 4), der humane Transferrinrezeptor (Tfr) 2β (SEQ ID NO. 1) oder die extrazelluläre Domäne des humanen Tfr2α (SEQ ID NO. 1), der murine Transferrinrezeptor (Tfr) 2β (SEQ ID NO. 2) oder die extrazelluläre Domäne des murinen Tfr2α (SEQ ID NO. 2) ist.

6. Fusionsprotein umfassend mindestens ein Protein nach einem der Ansprüche 1 bis 5 zur Verwendung in der Behandlung von primär oder sekundär sklerosierenden Erkrankungen.

7. Protein zur Verwendung nach einem der Ansprüche 1 bis 5 oder Fusionsprotein zur Verwendung nach Anspruch 6, **dadurch gekennzeichnet, dass** das Protein oder Fusionsprotein mindestens eine Modifizierung ausgewählt aus Proteinen enthaltend D-Aminosäuren, Pseudopeptidbindungen, Aminoalkoholen, nicht-proteinogenen Aminosäuren, Aminosäuren mit modifizierten Seitengruppen und/oder zirkularisierten Proteinen aufweist.

8. Verwendung eines Proteins nach einem der Ansprüche 1 bis 5 oder 7 oder eines Fusionsproteins nach Anspruch 6 oder 7 zur Diagnostik von Mitgliedern der TGF-ß/BMP-Familie, wobei die Diagnostik die Detektion von Mitgliedern der TGF-ß/BMP-Familie umfasst.

9. Verwendung eines Proteins nach einem der Ansprüche 1 bis 5 oder 7 oder eines Fusionsproteins nach Anspruch 6 oder 7 bei der *in vitro* Diagnostik von primär oder sekundär sklerosierenden Erkrankungen oder von Erkrankungen mit erhöhter BMP-Rezeptoraktivierung.

10. Verfahren für die *in vitro* oder *ex vivo* Diagnostik von primär oder sekundär sklerosierenden Erkrankungen oder von Erkrankungen mit erhöhter BMP-Rezeptoraktivierung unter Verwendung eines Proteins nach einem der Ansprüche 1 bis 5 oder 7 oder eines Fusionsproteins nach Anspruch 6 oder 7.

11. Protein nach einem der Ansprüche 1 bis 5 oder 7 oder Fusionsprotein nach Anspruch 6 oder 7 zur Verwendung in der Behandlung von Erkrankungen mit erhöhter BMP-Rezeptoraktivierung.

12. Nukleinsäure umfassend eine Sequenz gemäß SEQ ID NO. 8 oder SEQ ID NO. 9.

13. Vektor umfassend eine Nukleinsäure nach Anspruch 12.

14. Pharmazeutische Zusammensetzung umfassend mindestens ein Protein nach einem der Ansprüche 1 bis 5 oder 7 oder ein Fusionsprotein nach Anspruch 6 oder 7 zur Verwendung in der Behandlung von primär oder sekundär sklerosierenden Erkrankungen, oder zur Verwendung in der Behandlung von Erkrankungen mit erhöhter BMP-Rezeptoraktivierung.

15. Verwendung einer pharmazeutischen Zusammensetzung umfassend mindestens ein Protein nach einem der Ansprüche 1 bis 5 oder 7 oder ein Fusionsprotein nach Anspruch 6 oder 7 zur in vitro Diagnostik von primär oder sekundär sklerosierenden Erkrankungen.

16. Verwendung einer pharmazeutischen Zusammensetzung nach Anspruch 15, wobei die Diagnostik die Detektion von Mitgliedern der TGF-ß/BMP-Familie umfasst.

## Claims

1. Protein or fragment thereof having an amino acid sequence having at least 70% identity to sequence SEQ ID NO. 1 for use in the treatment of primary or secondary sclerosing diseases, wherein the protein or the fragment thereof binds members of the transforming growth factor-β (TGF-β)/bone morphogenetic protein family.

2. Protein comprising sequence SEQ ID NO. 1 or SEQ ID NO. 2 for use in the treatment of primary or secondary sclerosing diseases.

3. Protein for use according to Claim 1 or 2 having a length of 232 amino acids to 801 amino acids.

4. Protein for use according to any of Claims 1 to 3, **characterized in that** the protein is a transferrin receptor (Tfr) 2α, a transferrin receptor (Tfr) 2β or an extracellular domain of Tfr2α.

5. Protein for use according to any of Claims 1 to 4, **characterized in that** the protein is the human transferrin receptor (Tfr) 2α (SEQ ID NO. 3), the murine transferrin receptor (Tfr) 2α (SEQ ID NO. 4), the human transferrin receptor (Tfr) 2β (SEQ ID NO. 1) or the extracellular domain of the human Tfr2α (SEQ ID NO. 1), the murine transferrin receptor (Tfr) 2β (SEQ ID NO. 2) or the extracellular domain of the murine Tfr2α (SEQ ID NO. 2).

6. Fusion protein comprising at least one protein according to any of Claims 1 to 5 for use in the treatment of primary or secondary sclerosing diseases.

7. Protein for use according to any of Claims 1 to 5 or fusion protein for use according to Claim 6, **characterized in that** the protein or fusion protein has at least one modification selected from proteins containing D-amino acids, pseudopeptide bonds, amino alcohols, non-proteinogenic amino acids, amino acids having modified side groups and/or circularized proteins.

8. Use of a protein according to any of Claims 1 to 5 or 7 or of a fusion protein according to Claim 6 or 7 for the diagnosis of members of the TGF-β/BMP family, wherein the diagnosis comprises the detection of members of the TGF-β/BMP family.

9. Use of a protein according to any of Claims 1 to 5 or 7 or of a fusion protein according to Claim 6 or 7 in the in vitro diagnosis of primary or secondary sclerosing diseases or of diseases with increased BMP receptor activation.

10. Method for the in vitro or ex vivo diagnosis of primary or secondary sclerosing diseases or of diseases with increased BMP receptor activation using a protein according to any of Claims 1 to 5 or 7 or a fusion protein according to Claim 6 or 7.

11. Protein according to any of Claims 1 to 5 or 7 or fusion protein according to Claim 6 or 7 for use in the treatment of diseases with increased BMP receptor activation.

12. Nucleic acid comprising a sequence according to SEQ ID NO. 8 or SEQ ID NO. 9.

13. Vector comprising a nucleic acid according to Claim 12.

14. Pharmaceutical composition comprising at least one protein according to any of Claims 1 to 5 or 7 or a fusion protein according to Claim 6 or 7 for use in the treatment of primary or secondary sclerosing diseases, or for use in the treatment of diseases with increased BMP receptor activation.

15. Use of a pharmaceutical composition comprising at least one protein according to any of Claims 1 to 5 or 7 or a fusion protein according to Claim 6 or 7 for the in vitro diagnosis of primary or secondary sclerosing diseases.

16. Use of a pharmaceutical composition according to Claim 15, wherein the diagnosis comprises the detection of members of the TGF-β/BMP family.

## Revendications

1. Protéine ou fragment de celle-ci présentant une séquence d'acides aminés présentant au moins 70 % d'identité avec la séquence SEQ ID NO. 1, pour utilisation dans le traitement de maladies sclérosantes primaires ou secondaires, la protéine ou le fragment de celle-ci se liant à des membres de la famille du facteur de croissance transformant β (TGF-β)/des protéines morphogénétiques osseuses.

2. Protéine comprenant la séquence SEQ ID NO 1 ou la séquence SEQ ID NO. 2, pour utilisation dans le traitement de maladies sclérosantes primaires ou secondaires.

3. Protéine pour utilisation selon la revendication 1 ou 2, présentant une longueur de 232 acides aminés à 801 acides aminés.

4. Protéine pour utilisation selon l'une des revendications 1 à 3, **caractérisée en ce que** la protéine est un récepteur de transferrine (Tfr) 2α, un récepteur de transferrine (Tfr) 2β ou un domaine extracellulaire de Tfr2α.

5. Protéine pour utilisation selon l'une des revendications 1 à 4, **caractérisée en ce que** la protéine est le récepteur de la transferrine humain (Tfr) 2α (SEQ ID NO. 3), le récepteur de la transferrine murin (Tfr) 2α (SEQ ID NO. 4), le récepteur de la transferrine humain (Tfr) 2β (SEQ ID NO. 1) ou le domaine extracellulaire du Tfr2α humain (SEQ ID NO. 1), le récepteur de la transferrine murin (Tfr) 2β (SEQ ID NO. 2) ou le domaine extracellulaire du Tfr2α murin (SEQ ID NO. 2).

6. Protéine de fusion comprenant au moins une protéine selon l'une des revendications 1 à 5, pour utilisation dans le traitement de maladies sclérosantes primaires ou secondaires.

7. Protéine pour utilisation selon l'une des revendications 1 à 5 ou protéine de fusion pour utilisation selon la revendication 6, **caractérisée en ce que** la protéine ou la protéine de fusion présente au moins une modification choisie parmi les protéines contenant des acides aminés D, les liaisons pseudopeptidiques, les aminoalcools, les acides aminés non protéinogènes, les acides aminés présentant des groupes latéraux modifiés et/ou les protéines circularisées.

8. Utilisation d'une protéine selon l'une des revendications 1 à 5 ou 7 ou d'une protéine de fusion selon la revendication 6 ou 7 pour le diagnostic de membres de la famille TGF-β/BMP, le diagnostic comprenant la détection de membres de la famille TGF-β/BMP.

9. Utilisation d'une protéine selon l'une des revendications 1 à 5 ou 7 ou d'une protéine de fusion selon la revendication 6 ou 7 lors du diagnostic in vitro de maladies sclérosantes primaires ou secondaires ou de maladies présentant une activation accrue du récepteur BMP.

10. Procédé pour le diagnostic in vitro ou ex vivo de maladies sclérosantes primaires ou secondaires ou de maladies présentant une activation accrue du récepteur BMP utilisant une protéine selon l'une des revendications 1 à 5 ou 7 ou une protéine de fusion selon la revendication 6 ou 7.

11. Protéine selon l'une des revendications 1 à 5 ou 7 ou protéine de fusion selon la revendication 6 ou 7, pour utilisation dans le traitement de maladies présentant une activation accrue du récepteur BMP.

12. Acide nucléique comprenant une séquence selon SEQ ID NO. 8 ou SEQ ID NO. 9.

13. Vecteur comprenant un acide nucléique selon la revendication 12.

14. Composition pharmaceutique comprenant au moins une protéine selon l'une des revendications 1 à 5 ou 7 ou une protéine de fusion selon la revendication 6 ou 7, pour utilisation dans le traitement de maladies sclérosantes primaires ou secondaires ou pour utilisation dans le traitement de maladies présentant une activation accrue du récepteur BMP.

15. Utilisation d'une composition pharmaceutique comprenant au moins une protéine selon l'une des revendications 1 à 5 ou 7 ou une protéine de fusion selon la revendication 6 ou 7 pour le diagnostic in vitro de maladies sclérosantes primaires ou secondaires.

16. Utilisation d'une composition pharmaceutique selon la revendication 15, le diagnostic comprenant la détection de membres de la famille des TGF-β/BMP.
